# EUROPEAN PATENT APPLICATION

(11) **EP 4 759 895 A1**
(43) Date of publication of application: **17.06.2026**
(21) Application number: 24851337.6
(22) Date of filing: 10.05.2024
(51) Int. Cl.: C09K 11/08, B82Y 5/00, B82Y 40/00, C08K 3/08, C08K 3/36, C08L 5/08, C08L 101/00, C09K 11/59

(54) **FLUORESCENT PARTICLES AND METHOD FOR PRODUCING FLUORESCENT PARTICLES**

(30) Priority: 09.08.2023 JP 2023130134
(71) Applicant: TOKUYAMA CORPORATION, Yamaguchi 745-8648 (JP)
(72) Inventor: HARADA ICHIMARU, Hiroaki, Shunan-shi, Yamaguchi 745-8648 (JP); KIKUCHI, Shigetoshi, Shunan-shi, Yamaguchi 745-8648 (JP)
(74) Representative: Hoffmann Eitle
(86) International application number: PCT/JP2024/017363
(87) International publication number: WO 2025/032903

(57) **Abstract**

An object of an aspect of the present invention is to provide a fluorescent particle having superior dispersibility. A fluorescent particle (100) in accordance with an aspect of the present invention, includes a core particle (10A) charged with a first charge that is a positive charge or a negative charge, and further includes on the core particle: at least one polymer layer (12) that contains a polymer and is charged with a second charge that is a positive charge or a negative charge; and at least one fluorescent layer (11A, 11B) that contains fluorescent metal nanoclusters (13) and is charged with a third charge having a sign opposite to that of the second charge.

## Description

### Technical Field

The present invention relates to a fluorescent particle and a method for producing a fluorescent particle.

### Background Art

In the field of diagnostics using immunochromatography and the like, a gold colloid labeled with an antibody and exhibiting a color by absorbing light of a specific wavelength may be used to detect a target antigen. However, because the color exhibited by the gold colloid is weak, a method using the gold colloid may have insufficient detection sensitivity.

To improve detection sensitivity in diagnostics, fluorescent particles usable as an alternative to gold colloid have been developed. As such a technology, for example, Non-patent Literature 1 discloses fluorescent silica particles in which fluorescent gold nanoclusters are supported on porous silica particles via amide bonds.

### Citation List

### [Non-patent Literature]

[Non-patent Literature 1]
Y. Yuan, et al., Cell Prolif, 2021, 54, e13008.

### Summary of Invention

### Technical Problem

However, in the technology disclosed in Non-patent Literature 1, activated carboxy groups present on a protein that coats the gold nanoclusters form amide bonds not only with amino groups present on the surface of the porous silica particles but also with amino groups present on surrounding protein molecules, and thus the resulting fluorescent silica particles are prone to aggregation.

An object of an aspect of the present invention is to provide a fluorescent particle having superior dispersibility.

### Solution to Problem

To achieve the object, a fluorescent particle in accordance with an aspect of the present invention, includes a core particle charged with a first charge that is a positive charge or a negative charge, and further includes on the core particle: at least one polymer layer that contains a polymer and is charged with a second charge that is a positive charge or a negative charge; and at least one fluorescent layer that contains fluorescent metal nanoclusters and is charged with a third charge having a sign opposite to that of the second charge.

A method for producing a fluorescent particle in accordance with an aspect of the present invention, the method includes: at least one polymer layer formation step of forming, on a core particle charged with a first charge that is a positive charge or a negative charge, at least one polymer layer that contains a polymer and is charged with a second charge that is a positive charge or a negative charge; and at least one fluorescent layer formation step of forming, on the core particle, at least one fluorescent layer that contains fluorescent metal nanoclusters and is charged with a third charge having a sign opposite to that of the second charge.

### Advantageous Effects of Invention

According to an aspect of the present invention, it is possible to provide a fluorescent particle having superior dispersibility.

### Brief Description of Drawings

Fig. 1 is a schematic cross-sectional view of a fluorescent particle in accordance with an embodiment of the present invention.
Fig. 2 is a schematic cross-sectional view of a fluorescent particle in accordance with another embodiment of the present invention.
Fig. 3 is a graph showing fluorescence spectra of particles of Examples 1 and 2, and Reference Examples 1 and 2.
Fig. 4 is SEM images of the particles of Examples 1 and 2, and Reference Examples 1 and 2.
Fig. 5 is a graph showing particle diameter distributions of the particles of Examples 1 and 2 and Reference Examples 1 and 2.
Fig. 6 is a graph showing zeta potentials of the particles of Examples 1 and 2, and Reference Examples 1 and 2.
Fig. 7 is a graph showing a fluorescence spectrum of fluorescent particles of Example 3.
Fig. 8 is an SEM image of the fluorescent particles of Example 3.
Fig. 9 is a graph showing a fluorescence spectrum of fluorescent particles of Example 5.
Fig. 10 is an SEM image of the fluorescent particles of Example 5.

### Description of Embodiments

### [Fluorescent particle]

Hereinbelow, an embodiment of the present invention will be described in detail. As used herein, "A to B", unless otherwise specified, indicates a range of not less than A and not more than B. Where a plurality of numerical ranges are listed in this specification, a new numerical range formed by combining an upper limit of one numerical range with a lower limit of another numerical range is also considered to be described herein.

A fluorescent particle in accordance with an aspect of the present invention, includes a core particle charged with a first charge that is a positive charge or a negative charge, and further includes on the core particle: at least one polymer layer that contains a polymer and is charged with a second charge that is a positive charge or a negative charge; and at least one fluorescent layer that contains fluorescent metal nanoclusters and is charged with a third charge having a sign opposite to that of the second charge.

### [Core particle]

In an aspect of the present invention, the core particle may be any inorganic particle, organic particle, or organic-inorganic composite particle, but is not limited thereto. Examples of the inorganic particles include: metal oxide particles such as silica particles and titanium oxide particles; metal particles such as gold particles, silver particles, and platinum particles; and alloy particles formed by combining a plurality of types of metals. Examples of the organic particles include: polymer particles such as polystyrene particles and polymethacrylic acid particles; and semiconductor particles.

From the viewpoint of more easily supporting the fluorescent layer and the polymer layer on the core particle, the core particle is preferably selected from the group consisting of metal oxide particles, metal particles, and polymer particles, and more preferably selected from the group consisting of silica particles, polymer particles, and metal particles. Further, from the viewpoint of reducing a wavelength shift in the fluorescence wavelength of the metal nanoclusters and allowing the resulting fluorescent particle to exhibit the intrinsic fluorescence wavelength of the metal nanoclusters, the core particle is more preferably a silica particle.

The core particle is charged with a first charge that is a positive charge or a negative charge. The core particle may have a cationic functional group or an anionic functional group on its surface, and may be charged with the first charge by the functional group. Alternatively, the core particle may be doped with a cation, an anion, or an electron, and may be charged with the first charge by the cation, the anion, or the electron.

Typically, when the core particle has a cationic functional group on its surface, the first charge is a positive charge, and when the core particle has an anionic functional group on its surface, the first charge is a negative charge. Examples of the cationic functional groups include: amine groups such as primary amine groups (i.e., amino groups), secondary amine groups, tertiary amine groups, and quaternary amine groups (i.e., ammonium cations); and phosphonium groups (i.e., phosphonium cations). Examples of the anionic functional groups include: a carboxy group; and a sulfo group. Among these, from the viewpoint of stabilizing the support of the fluorescent layer on the core particle, the core particle preferably has, on a surface thereof, a functional group selected from the group consisting of an amine group and a phosphonium group.

The average particle diameter of the core particle may be appropriately selected according to the application and the like of the fluorescent particles, and is not particularly limited, but may be, for example, not less than 100 nm and not more than 100 µm, not less than 300 nm and not more than 50 µm, or not less than 500 nm and not more than 10 µm. As used herein, the average particle diameter refers to the average particle diameter calculated using an SEM image.

### [Polymer layer]

The polymer layer contains a polymer, and may, for example, contain the polymer as a main component. As used herein, the main component refers to a component that accounts for not less than 50% by mass in the polymer layer.

Examples of the polymers contained in the polymer layer include cationic polymers and anionic polymers. Examples of the cationic polymers include: amino group-containing polymers such as chitosan and polylysine; and quaternary amine group-containing polymers such as sulfobetaine polymers. Examples of the anionic polymers include: sulfo group-containing polymers such as polystyrene sulfonic acid; and carboxy group-containing polymers such as alginic acid.

The polymer may be classified based on the main backbone of the polymer, in addition to the classification by ionicity described above. For example, examples of the polymer include: polysaccharide polymers such as chitosan and alginic acid; olefin-based polymers such as polyacrylic acid and polystyrene sulfonic acid; and proteins such as gelatin and polylysine. Regarding polylysine, it is not limited to polylysine made of 50 or more molecules of lysine, which is classified as a protein, and polylysine made of 49 or fewer molecules of lysine, which is therefore classified as a peptide in this specification, may also be used as the polymer.

From the viewpoint of increasing the coverage of the core particle by the polymer layer and stabilizing the support of the polymer layer on the core particle by entanglement between polymer chains, the weight-average molecular weight of the polymer is preferably not less than 1,000, and more preferably not less than 5,000. Further, from the viewpoint of further improving the dispersibility of the fluorescent particles, the weight-average molecular weight of the polymer is preferably not more than 1,000,000, and more preferably not more than 500,000. Herein, the weight-average molecular weight of the polymer is determined by a gel permeation chromatography method.

From the viewpoint of reducing a wavelength shift in the fluorescence wavelength of the metal nanoclusters, the polymer contained in the polymer layer is preferably a polymer that does not contain an aromatic ring in its repeating unit, more preferably is at least one selected from the group consisting of chitosan and polylysine, and even more preferably is chitosan.

The polymer layer may further contain an additive such as a counter ion for an anion or a cation possessed by the polymer contained in the polymer layer.

The polymer layer is charged with a second charge that is a positive charge or a negative charge. The second charge may be determined by the type of polymer contained in the polymer layer. Typically, when the polymer layer contains a cationic polymer, the second charge is a positive charge, and when the polymer layer contains an anionic polymer, the second charge is a negative charge.

### [Fluorescent layer]

The fluorescent layer contains fluorescent metal nanoclusters. As used herein, a metal nanocluster is a particle formed by aggregation of at least one selected from the group consisting of metal atoms and metal oxide molecules. In an aspect of the present invention, the metal nanocluster may be any fluorescent metal nanocluster, and is not particularly limited. The total number of the atoms and the molecules forming the metal nanocluster may be, for example, not less than 2 and not more than 1000, and from the viewpoint of further improving fluorescence intensity, is preferably not less than 5 and not more than 100, and more preferably not less than 10 and not more than 50. The average particle diameter of the metal nanoclusters may be, for example, not less than 0.5 nm and not more than 10 nm.

Examples of the metal atoms forming the metal nanocluster include gold, silver, copper, platinum, palladium, iridium, and iron. The metal nanocluster may be composed of a single type of metal atom, or may be composed of a combination of a plurality of types of metal atoms. The metal atom may be appropriately selected according to the desired fluorescence intensity, fluorescence wavelength, and biotoxicity depending on the application of the fluorescent particles. From the viewpoint of facilitating the synthesis of metal nanoclusters having superior fluorescence, the metal atom is preferably at least one selected from the group consisting of noble metals, and more preferably is at least one selected from the group consisting of gold and silver. In other words, the metal nanocluster is more preferably a nanocluster containing gold, silver, or both.

Examples of the metal oxide molecules forming the metal nanocluster include silica, alumina, titanium oxide, and iron oxide.

In an aspect of the present invention, the metal nanoclusters are preferably coated with a coating agent. The coating agent is a compound that binds to the metal atoms forming the metal nanoclusters. The coating agent protects the metal nanoclusters from further aggregation of metal atoms and reduces excessive growth of the metal nanoclusters. Further, the coating agent may also exhibit a function of imparting a third charge to the metal nanoclusters, and the metal nanoclusters coated with such a coating agent are suitable for a layered structure formed using electrostatic interaction.

The coating agent has a functional group capable of binding to a metal atom, and may be, for example, a thiol group-containing compound. Further, from the viewpoint of coating the metal nanoclusters while maintaining dispersibility in an aqueous phase, the coating agent may typically be hydrophilic. Examples of the coating agents include, but are not limited to: amino acids such as cysteine and histidine; peptides such as glutathione; proteins such as bovine serum albumin (BSA), gelatin, and trypsin; aromatic ring-containing compounds such as 4-mercaptobenzoic acid (p-MBA), 2-mercaptobenzoic acid (m-MBA), and captopril (Capt); and fatty chain-containing compounds such as 11-mercaptoundecanoic acid (11-MUA). As used herein, a peptide refers to a compound formed by bonding 2 to 49 amino acid molecules, and a protein refers to a compound formed by bonding 50 or more amino acid molecules.

From the viewpoints of high biocompatibility, easy availability, and a large effect of imparting a negative charge as the third charge to the metal nanoclusters, the coating agent is preferably at least one selected from the group consisting of amino acids, peptides, and proteins. Among these, to enable a reduction reaction in a mild environment and suppression of particle aggregation, the coating agent is more preferably a protein or a peptide, and even more preferably is BSA or glutathione.

The fluorescent layer is charged with the third charge having a sign opposite to that of the second charge. Here, the third charge only needs to have a sign opposite to that of the second charge, and the absolute value of the charge amount of the third charge is not particularly limited.

### [Layered structure of fluorescent particle]

The fluorescent particle may have a layered structure in which at least one polymer layer and at least one fluorescent layer are layered on the core particle. Here, it is preferable that the polymer layer and the fluorescent layer are arranged on the core particle such that the charges of adjacent layers and particle have opposite signs to each other. For example, when the charge of the core particle (the first charge) is a charge having the same sign as the charge of the polymer layer (the second charge), one fluorescent layer charged with the third charge, which is a charge having a sign opposite to that of the first charge and the second charge, is directly arranged on the core particle, and one polymer layer is arranged on the surface of the fluorescent layer facing away from the core particle. In other words, when the first charge is a charge having the same sign as the second charge, it is preferable that the fluorescent particle has at least one fluorescent layer disposed between the core particle and the polymer layer.

In the fluorescent particle, the number of fluorescent layers may be one, or may be two or more, and is preferably two or more from the viewpoint of further improving the fluorescence intensity. When providing two or more fluorescent layers, a polymer layer may be provided between two fluorescent layers, and the polymer layers and the fluorescent layers may be alternately layered.

When the number of fluorescent layers is two or more, the composition of the metal nanoclusters contained in each fluorescent layer may be the same or different. For example, the fluorescent properties of the fluorescent particle as a whole may be adjusted by varying the composition of the metal nanoclusters contained in each fluorescent layer.

The numbers of polymer layers and fluorescent layers included in the fluorescent particle may be the same or different. Here, in the fluorescent particle, a desired fluorescence intensity can be obtained by appropriately setting the number of fluorescent layers. For example, the fluorescent particle may have at least two fluorescent layers from the viewpoint of further improving the fluorescence intensity.

The outermost layer among all the polymer layers and fluorescent layers included in the fluorescent particle may be either a polymer layer or a fluorescent layer. For example, the fluorescent particle may have at least one fluorescent layer disposed outside the outermost polymer layer. According to such a layered structure, the fluorescence intensity of the fluorescent particle is further improved because of the fluorescent layer that is the outermost layer among all the polymer layers and fluorescent layers. Also, for example, the fluorescent particle may have at least one polymer layer disposed outside the outermost fluorescent layer. According to such a layered structure, the dispersibility of the fluorescent particles is further improved because the fluorescent particles repel each other because of the second charge provided by the polymer layer that is the outermost layer among all the polymer layers and fluorescent layers.

The fluorescent particle may have an additional layer in addition to the at least one polymer layer and the at least one fluorescent layer described above. Examples of the additional layer include a base layer and a protective layer. There is no particular limitation on the arrangement of the additional layer in the layered structure of the fluorescent particle, and the additional layer may be arranged inside or outside all of the at least one polymer layer and the at least one fluorescent layer, or may be arranged between one polymer layer and one fluorescent layer. For example, a base layer may be arranged inside all of the at least one polymer layer and the at least one fluorescent layer (i.e., on the core particle side). Also, for example, a protective layer may be arranged outside all of the at least one polymer layer and the at least one fluorescent layer (i.e., on the side opposite to the core particle side).

Each of the at least one polymer layer and the at least one fluorescent layer included in the fluorescent particle, and the additional layer if included, preferably covers the entire surface of the core particle directly or indirectly, but is not limited thereto, and only needs to cover at least a part of the core particle. The dispersibility of the fluorescent particles imparted by the at least one polymer layer and the fluorescence of the fluorescent particles imparted by the at least one fluorescent layer are more strongly expressed as the coverage (the ratio of the surface area covered by each layer to the total surface area of the core particle) of each layer on the core particle increases. The coverage of each layer can be, for example, not less than 10%, not less than 20%, not less than 30%, not less than 40%, not less than 50%, not less than 60%, not less than 70%, not less than 80%, not less than 90%, or not less than 100%, and is preferably 100%. In an aspect of the present invention, the coverage can be controlled by changing the respective amounts of the polymer and the metal nanoclusters added to a solution, and by changing the contact time between the core particle and the solution, in the method for producing the fluorescent particle.

### [Properties of fluorescent particles]

The fluorescent properties of the fluorescent particles are not particularly limited. For example, an intensity peak in a fluorescence spectrum upon excitation with excitation light in a wavelength range of 200 nm to 1000 nm may appear within a wavelength range of 400 nm to 2000 nm. To provide fluorescent particles having a fluorescence wavelength that facilitates the identification of the fluorescence from the fluorescent particles in a biological sample, the intensity peak is preferably within a wavelength range of 600 nm to 1500 nm.

From the viewpoint of improving the ease of handling of the fluorescent particles, the average particle diameter of the fluorescent particles is preferably not less than 100 nm, more preferably not less than 200 nm, and even more preferably not less than 300 nm. Further, from the viewpoint of reducing optical scattering by the fluorescent particles, the average particle diameter of the fluorescent particles is preferably not more than 100 µm, more preferably not more than 50 µm, and even more preferably not more than 1000 nm.

The fluorescent particle in accordance with an aspect of the present invention has superior dispersibility. Here, the dispersibility may be evaluated by observing an SEM image or the like, or may be expressed using a ratio of the average particle diameter of the fluorescent particles to the average particle diameter of the core particles. It can be considered that the lower the ratio, the closer the average particle diameter of the fluorescent particles is to that of the core particles, which is the raw material, and thus the less aggregation of the core particles occurs during the production process of the fluorescent particles. The ratio of the average particle diameter of the fluorescent particles to the average particle diameter of the core particles is preferably not more than 2.0, more preferably not more than 1.5, and even more preferably not more than 1.25.

The shape of the fluorescent particle is not particularly limited, and may be, for example, substantially spherical, plate-like, or rod-like. Among these, from the viewpoint of the ease of handling, the shape of the fluorescent particle is preferably substantially spherical, and more preferably spherical. The shape of the fluorescent particle can be adjusted by appropriately selecting the shape of the core particle used for production, and for example, by using substantially spherical core particles, substantially spherical fluorescent particles can be produced.

From the viewpoint of further improving the dispersion stability of the fluorescent particles, the zeta potential of the fluorescent particle surface is preferably not less than 10 mV, and more preferably not less than 20 mV. Alternatively, from the viewpoint of further improving the dispersion stability of the fluorescent particles, the zeta potential of the fluorescent particle surface is preferably not more than -10 mV, and more preferably not more than -20 mV. Herein, the zeta potential is determined by an electrophoresis method.

### [Fluorescent particle in accordance with embodiment]

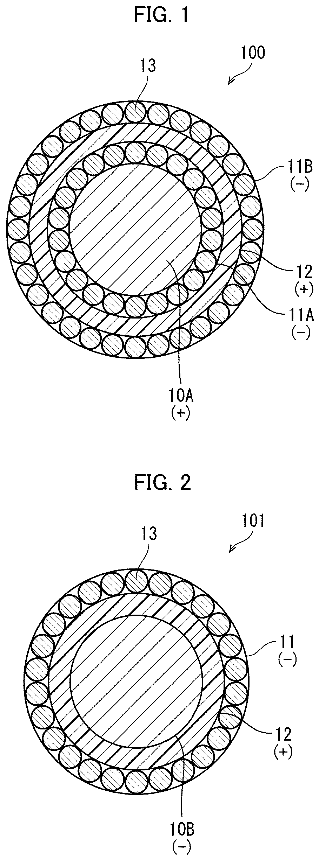

Hereinbelow, a fluorescent particle in accordance with an embodiment of the present invention will be described with reference to Fig. 1. Fig. 1 is a schematic cross-sectional view of a fluorescent particle 100 in accordance with an embodiment of the present invention. The fluorescent particle 100 has a positively charged core particle 10A, a negatively charged fluorescent layer 11A, a positively charged polymer layer 12, and a negatively charged fluorescent layer 11B. The fluorescent layer 11A, the polymer layer 12, and the fluorescent layer 11B are layered in this order on the core particle 10A. In the present embodiment, the core particle 10A is a silica particle, and is positively charged by amino groups present on its surface. Each of the fluorescent layers 11A and 11B contains fluorescent metal nanoclusters 13. The metal nanoclusters 13 are coated with BSA, and are thereby negatively charged. The polymer layer 12 contains chitosan, and is positively charged by amino groups in chitosan. According to the present embodiment, a fluorescent particle 100 having both fluorescence and dispersibility is provided. Further, due to the fluorescent layer 11B as the outermost layer, the fluorescent particle 100 exhibits stronger fluorescence intensity. Further, because the silica particle that is the core particle 10A is less likely to shift the fluorescence wavelength of the metal nanoclusters 13, the fluorescent particle 100 can exhibit the intrinsic fluorescence wavelength of the metal nanoclusters 13.

A fluorescent particle in accordance with another embodiment of the present invention will be described with reference to Fig. 2. Fig. 2 is a schematic cross-sectional view of a fluorescent particle 101 in accordance with an embodiment of the present invention. The fluorescent particle 101 has a negatively charged core particle 10B, and also has a positively charged polymer layer 12 and a negatively charged fluorescent layer 11 that are layered in this order on the core particle 10B. In the present embodiment, the core particle 10B is a silica particle, and is negatively charged by carboxy group present on its surface. The fluorescent layer 11 contains the fluorescent metal nanoclusters 13.

As understood from the fluorescent particles 100 and 101 illustrated in Figs. 1 and 2, respectively, in an aspect of the present invention, the layering order of the core particle, the polymer layer, and the fluorescent layer can be appropriately selected according to the charge possessed by each member.

### [Method for producing fluorescent particle]

Hereinbelow, another aspect of the present invention will be described. For convenience of explanation, the description of members having the same functions as the members described above will not be repeated.

A method for producing a fluorescent particle in accordance with an aspect of the present invention includes: at least one polymer layer formation step of forming, on a core particle charged with a first charge that is a positive charge or a negative charge, at least one polymer layer that contains a polymer and is charged with a second charge that is a positive charge or a negative charge; and at least one fluorescent layer formation step of forming, on the core particle, at least one fluorescent layer that contains fluorescent metal nanoclusters and is charged with a third charge having a sign opposite to that of the second charge.

### [Charging step]

The method for producing a fluorescent particle may further include, before the polymer layer formation step and the fluorescent layer formation step, a charging step of obtaining the core particle by a charging treatment. The charging step can be performed, for example, by subjecting an uncharged core particle raw material to a charging treatment. Here, examples of the charging treatments include, but are not limited to: a chemical treatment of modifying the surface of the raw material with a cationic functional group or an anionic functional group; and a physical treatment of subjecting the raw material to an electron beam irradiation method or an ion implantation method.

### [Polymer layer formation step]

The polymer layer formation step is a step of forming at least one polymer layer on the core particle. The polymer layer formation step can be performed, for example, by bringing the core particle into direct or indirect contact with a solution containing a polymer. Here, one or both of the fluorescent layer and the additional layer may be formed in advance on the core particle, and in such a case, by bringing the layer into contact with the polymer solution, the core particle may thereby be indirectly contacted with the polymer solution to perform the polymer layer formation step. For the contact, methods known in the art such as spraying, coating, and dipping can be used.

The solvent of the polymer solution may be appropriately selected according to the type of polymer, and examples thereof include water. In the polymer solution, the polymer concentration may be not less than 1 mg/L, and is preferably not less than 10 mg/L. Further, in the polymer solution, the polymer concentration may be not more than 100 g/L, and is preferably not more than 10 g/L.

In the polymer layer formation step, the amount of polymer used (the amount of polymer contained in the polymer solution used) may be not less than 1 mg, and is preferably not less than 10 mg, per 1 g of the core particles used. The amount of polymer used may be not more than 100 mg, and is preferably not more than 50 mg, per 1 g of the core particles used.

In the polymer layer formation step, the contact time between the core particle and the polymer solution may be, for example, not less than 0.5 hours and not more than 10 hours, or may be not less than 1 hour and not more than 5 hours. Further, the contact temperature is preferably not less than 0 °C and not more than 100 °C, and more preferably not less than 20 °C and not more than 50 °C. According to such a preferable contact temperature, denaturation of the coating agent coating the metal nanoclusters, for example, a protein, can be prevented, and therefore, changes in the fluorescent properties and particle diameter distribution of the metal nanoclusters due to denaturation can be prevented.

### [Fluorescent layer formation step]

The fluorescent layer formation step is a step of forming at least one fluorescent layer on the core particle. The fluorescent layer formation step can be performed, for example, by bringing the core particle into direct or indirect contact with a dispersion solution of the metal nanoclusters. Here, one or both of the polymer layer and the additional layer may be formed in advance on the core particle, and in such a case, by bringing the layer into contact with the dispersion solution, the core particle may be brought into indirect contact with the dispersion solution to perform the fluorescent layer formation step. For the contact, methods known in the art such as spraying, coating, and dipping can be used.

Examples of the solvents for the dispersion solution include water. In the dispersion solution, the concentration of the metal nanoclusters may be not less than 0.25 g/L, and is preferably not less than 0.5 g/L. Further, in the dispersion solution, the concentration of the metal nanoclusters may be not more than 5 g/L, and is preferably not more than 1 g/L.

In the fluorescent layer formation step, the amount of metal nanoclusters used (the amount of metal nanoclusters contained in the dispersion solution used) may be not less than 1 mg, and is preferably not less than 5 mg, per 1 g of the core particles used. The amount of metal nanoclusters used may be not more than 50 mg, and is preferably not more than 25 mg, per 1 g of the core particles used.

In the fluorescent layer formation step, the contact time between the core particle and the dispersion solution may be, for example, not less than 0.5 hours and not more than 10 hours, or may be not less than 1 hour and not more than 5 hours. Further, the contact temperature is preferably not less than 0 °C and not more than 100 °C, and more preferably not less than 20 °C and not more than 50 °C. According to such a preferable contact temperature, denaturation of the coating agent coating the metal nanoclusters, for example, a protein, can be prevented, and therefore, changes in the fluorescent properties and particle diameter distribution of the metal nanoclusters due to denaturation can be prevented.

### [Set of steps]

The method for producing a fluorescent particle only needs to include at least one polymer layer formation step and at least one fluorescent layer formation step. By performing each of these steps at least once, it is possible to obtain a fluorescent particle. Further, in the production method, one or both of the steps may be performed twice. For example, the production method may include a step in which a set of steps, including one polymer layer formation step and one fluorescent layer formation step, is repeated at least twice. Here, in the step of repeating the set of steps at least twice, the polymer layer formation step and the fluorescent layer formation step are performed alternately. According to such a configuration, it is possible to obtain a fluorescent particle in which two or more polymer layers and two or more fluorescent layers are alternately formed by a Layer-By-Layer (LBL) method utilizing electrostatic interaction between the second charge and the third charge.

In the method for producing a fluorescent particle, the polymer layer formation steps and the fluorescent layer formation steps are typically performed alternately, but it is not particularly limited which step is performed first. The step performed first may be selected according to the charge of the core particle. For example, when the charge of the core particle (the first charge) has the same sign as the charge of the polymer layer (the second charge), it is preferable that the production method first include the fluorescent layer formation step between the polymer layer formation step and the fluorescent layer formation step; that is, that the production method include at least one fluorescent layer formation step before the polymer layer formation step.

The method for producing a fluorescent particle may include a step of forming an additional layer. The step of forming an additional layer can be performed using a method known in the art, according to the type of the additional layer and the desired arrangement of the additional layer.

Aspects of the present invention can also be expressed as follows:
A fluorescent particle in accordance with Aspect 1 of the present invention, includes a core particle charged with a first charge that is a positive charge or a negative charge, and further includes on the core particle: at least one polymer layer that contains a polymer and is charged with a second charge that is a positive charge or a negative charge; and at least one fluorescent layer that contains fluorescent metal nanoclusters and is charged with a third charge having a sign opposite to that of the second charge.

A fluorescent particle in accordance with Aspect 2 of the present invention is configured so that, in Aspect 1, at least one of the at least one fluorescent layer is disposed outside an outermost layer of the at least one polymer layer.

A fluorescent particle in accordance with Aspect 3 of the present invention is configured so that, in Aspect 1, at least one of the at least one polymer layer is disposed outside an outermost layer of the at least one fluorescent layer.

A fluorescent particle in accordance with Aspect 4 of the present invention is configured so that, in any one of Aspects 1 to 3, the first charge is a charge having the same sign as the second charge, and at least one of the at least one fluorescent layer is disposed between the core particle and the at least one polymer layer.

A fluorescent particle in accordance with Aspect 5 of the present invention is configured so that, in any one of Aspects 1 to 4, the first charge is a positive charge, and the core particle has, on a surface thereof, a functional group selected from the group consisting of an amine group and a phosphonium group.

A fluorescent particle in accordance with Aspect 6 of the present invention is configured so that, in any one of Aspects 1 to 5, the core particle is selected from the group consisting of a silica particle, a polymer particle, and a metal particle.

A fluorescent particle in accordance with Aspect 7 of the present invention is configured so that, in any one of Aspects 1 to 6, the fluorescent particle has an average particle diameter of not less than 100 nm and not more than 100 µm.

A fluorescent particle in accordance with Aspect 8 of the present invention is configured so that, in any one of Aspects 1 to 7, the polymer includes chitosan.

A fluorescent particle in accordance with Aspect 9 of the present invention is configured so that, in any one of Aspects 1 to 8, the metal nanoclusters are coated with a coating agent, and the coating agent is at least one selected from the group consisting of an amino acid, a peptide, and a protein.

A method for producing a fluorescent particle in accordance with Aspect 10 of the present invention, includes: at least one polymer layer formation step of forming, on a core particle charged with a first charge that is a positive charge or a negative charge, at least one polymer layer that contains a polymer and is charged with a second charge that is a positive charge or a negative charge; and at least one fluorescent layer formation step of forming, on the core particle, at least one fluorescent layer that contains fluorescent metal nanoclusters and is charged with a third charge having a sign opposite to that of the second charge.

A method for producing a fluorescent particle in accordance with Aspect 11 of the present invention is configured so that, in Aspect 10, the first charge is a charge having the same sign as the second charge, and at least one of the at least one fluorescent layer formation step is performed before the at least one polymer layer formation step.

A method for producing a fluorescent particle in accordance with Aspect 12 of the present invention is configured, in Aspect 10 or 11, to further include, before the at least one polymer layer formation step and the at least one fluorescent layer formation step, a charging step of obtaining the core particle by a charging treatment.

A method for producing a fluorescent particle in accordance with Aspect 13 of the present invention is configured so that, in any one of Aspects 10 to 12, the at least one polymer layer formation step and the at least one fluorescent layer formation step are each performed at not less than 0 °C and not more than 100 °C.

### [Additional remarks]

The present invention is not limited to the embodiments, but can be altered by a skilled person in the art within the scope of the claims. The present invention also encompasses, in its technical scope, any embodiment derived by combining technical means disclosed in differing embodiments.

### Examples

An example of the present invention will be described below. In the present example, fluorescent silica particles were produced using the LBL method.

### [Reference Example 1] Preparation of single-layer supported fluorescent silica particles (NH₂-silica/BSA-Au NCs particles)

An aqueous solution was prepared to have final concentrations of 0.36 mM BSA, 4.77 mM chloroauric acid (HAuCl₄), and 47.6 mM sodium hydroxide (NaOH). Next, the aqueous solution was stirred overnight at 37 °C to obtain an aqueous dispersion of BSA-coated gold nanoclusters (BSA-Au NCs) (gold atom concentration: 4.77 mM). As core particles, silica particles (NH₂-silica) (average particle diameter: 325 nm, surface functional group: amino group) were used. 0.25 mL of an aqueous dispersion of silica particles (particle concentration: 0.1 g/mL) was added to a 1.5 mL Eppendorf tube. Next, 0.25 mL of the aqueous dispersion of BSA-coated gold nanoclusters with a gold atom concentration of 4.77 mM was added to the aqueous dispersion of silica particles, and the mixture was stirred for 1 hour at room temperature using a shaker (MT-400, manufactured by Tomy Medico Co., Ltd.). The resulting solution was washed three times (3000 g, 5 min, 4 °C) using a centrifuge (MDX-110, manufactured by Tomy Kogyo Co., Ltd.), and the residue was redispersed in 0.25 mL of ultrapure water to obtain an aqueous dispersion of single-layer supported fluorescent particles of silica particle/fluorescent layer.

### [Example 1] Preparation of two-layer supported fluorescent silica particles (NH₂-silica/BSA-Au NCs/CS particles)

0.25 mL of a chitosan (CS) (FUJIFILM Wako Pure Chemical Corporation) solution (chitosan concentration: 1 mg/mL, 10 v/v% aqueous acetic acid solution) was prepared and added to the aqueous dispersion obtained in Reference Example 1. The mixture was stirred for 1 hour at room temperature using a shaker (MT-400, manufactured by Tomy Medico Co., Ltd.). The resulting solution was washed three times (3000 g, 5 min, 4 °C) using a centrifuge (MDX-110, manufactured by Tomy Kogyo Co., Ltd.), and the residue was redispersed in 0.25 mL of ultrapure water to obtain an aqueous dispersion of two-layer supported fluorescent silica particles of silica particle/fluorescent layer/polymer layer.

### [Example 2] Preparation of three-layer supported fluorescent silica particles (NH₂-silica/2BSA-Au NCs/CS particles)

0.25 mL of an aqueous dispersion of BSA-coated gold nanoclusters (gold atom concentration: 4.77 mM, prepared by the same method as in Reference Example 1) was added to the aqueous dispersion obtained in Example 1. The mixture was stirred for 1 hour at room temperature using a shaker (MT-400, manufactured by Tomy Medico Co., Ltd.). The resulting solution was washed three times (3000 g, 5 min, 4 °C) using a centrifuge (MDX-110, manufactured by Tomy Kogyo Co., Ltd.), and the residue was redispersed in 0.25 mL of ultrapure water to obtain an aqueous dispersion of three-layer supported fluorescent silica particles of silica particle/fluorescent layer/polymer layer/fluorescent layer.

### [Fluorescence spectrum measurement]

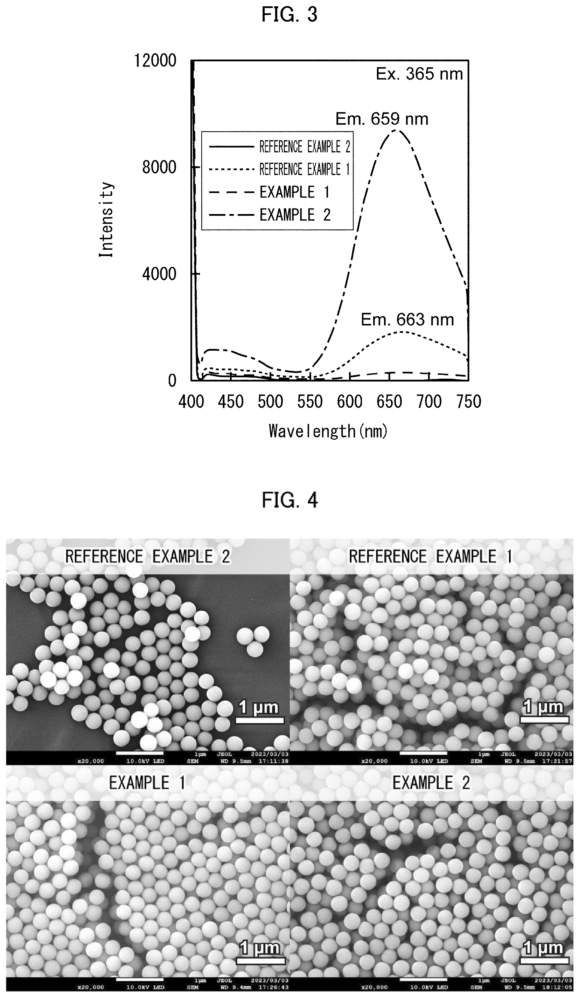

Fluorescence spectra were measured by a spectrofluorometer NanoDrop^{™} 3300 (Thermo Fisher Scientific Inc.) (excitation wavelength: 365 nm), using the aqueous dispersions of the fluorescent particles obtained in Reference Example 1, Example 1, and Example 2, and the aqueous dispersion of the silica particles used in Reference Example 1 (hereinafter, may be referred to as "Reference Example 2"). The measurement results of the fluorescence spectra are shown in Fig. 3.

As shown in Fig. 3, the fluorescent particles of Examples 1 and 2 exhibited fluorescence. Also, the three-layer supported fluorescent particles of fluorescent layer/polymer layer/fluorescent layer, obtained in Example 2, exhibited a fluorescence intensity approximately 5 times that of the single-layer supported fluorescent particles of Reference Example 1. This demonstrated that a layered structure having a fluorescent layer as the outermost layer contributes to higher fluorescence intensity.

Also, as shown in Fig. 3, the intensity peaks in the fluorescence spectra of Examples 1 and 2 appeared at 663 nm and 659 nm, respectively, which were close to the intensity peak originally exhibited by the BSA-coated gold nanoclusters (667 nm). From this, it was found that by using silica particles as the core particles, the fluorescence wavelength intrinsically possessed by the metal nanoclusters can be imparted to the fluorescent particles.

### [Observation of particle shape]

The shapes of the silica particles of Reference Example 2, and the fluorescent particles of Reference Example 1, Example 1, and Example 2 were observed using an electron microscope. SEM images were acquired using an electron microscope JSM-7800F Prime (JEOL Ltd.) under the conditions of an acceleration voltage of 10.0 kV, a WD of 9.5 mm, and a magnification of 20,000x. The acquired SEM images are shown in Fig. 4.

As shown in Fig. 4, the fluorescent particles of Examples 1 and 2 had a spherical shape similar to the silica particles of Reference Example 2, which were the core particles. From this, it was found that the layered structure formed by the LBL method does not affect the shape of the resulting fluorescent particles, and therefore, fluorescent particles having a shape similar to the core particles and having superior dispersibility can be obtained.

### [Particle diameter distribution determination]

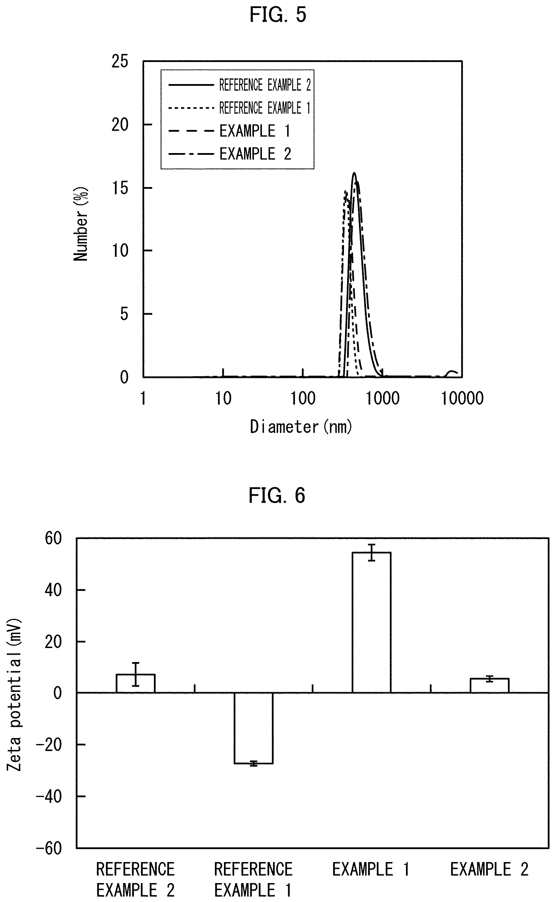

The particle diameter distributions of the silica particles of Reference Example 2, and the fluorescent particles of Reference Example 1, Example 1, and Example 2 were determined using a dynamic light scattering (DLS) method. The determination was performed using a zeta potential/particle size/molecular weight analyzer ELSZ-2000 (Otsuka Electronics Co., Ltd.). The determination results are shown in Fig. 5.

As shown in Fig. 5, the particles had a similar particle diameter distribution. From this, it can be seen that the fluorescent particles of Examples 1 and 2 have superior dispersibility, that aggregation of the particles is suppressed during the production process, and that the dispersion state derived from the raw material silica particles (the silica particles of Reference Example 2) is sufficiently maintained.

Also, the average particle diameter was determined using the SEM images acquired in the observation of particle shape. The average particle diameter was determined by randomly selecting five particles from within an SEM image and calculating the average particle diameter of these five particles. The average particle diameter of the silica particles of Reference Example 2 was 327 nm, the average particle diameter of the fluorescent particles of Reference Example 1 was 327 nm, the average particle diameter of the fluorescent particles of Example 1 was 327 nm, and the average particle diameter of the fluorescent particles of Example 2 was 347 nm. The determination results of the average particle diameter also showed that the fluorescent particles of Examples 1 and 2 suitably maintained the average particle diameter of the raw material silica particles, and therefore had superior dispersibility.

### [Zeta potential measurement of particles]

The zeta potentials of the silica particles of Reference Example 2, and the fluorescent particles of Reference Example 1, Example 1, and Example 2 were measured. The measurement was performed using a zeta potential/particle size/molecular weight analyzer ELSZ-2000 (Otsuka Electronics Co., Ltd.). The measurement results are shown in Fig. 6.

As shown in Fig. 6, the zeta potential alternately increases and decreases in the order of the silica particles of Reference Example 2, the fluorescent particles of Reference Example 1, the fluorescent particles of Example 1, and the fluorescent particles of Example 2. From this, it can be seen that a layered structure including the negatively charged fluorescent layers and the positively charged polymer layers is suitably formed. Also, from the fact that the zeta potential of the fluorescent particles of Example 1 was greater in the positive direction than the zeta potential of the silica particles of Reference Example 2, and from the fact that, as shown in Fig. 3, the fluorescence intensity of the fluorescent particles of Example 2 was much higher than the fluorescence intensity of the fluorescent particles of Reference Example 1, it was suggested that the greater the absolute value of the zeta potential, the higher the supported amount of a layer further formed thereon.

### [Example 3] Preparation of three-layer supported fluorescent silica particles (NH₂-silica/BSA-Au NCs/CS/GSH-Au NCs particles)

An aqueous solution was prepared to have final concentrations of 6.8 mM glutathione (GSH), 4.5 mM chloroauric acid (HAuCl₄), and 90 mM sodium hydroxide (NaOH). Next, the aqueous solution was stirred overnight at 37 °C to obtain an aqueous dispersion of GSH-coated gold nanoclusters (GSH-Au NCs) (gold atom concentration: 5 mM).

0.25 mL of the aqueous dispersion of GSH-coated gold nanoclusters (gold atom concentration: 5.0 mM) was added to an aqueous dispersion of NH₂-silica/BSA-Au NCs/CS particles obtained by the same method as in Example 1. The mixture was stirred for 1 hour at room temperature using a shaker (MT-400, manufactured by Tomy Medico Co., Ltd.). The resulting solution was washed three times (3000 g, 5 min, 4 °C) using a centrifuge (MDX-110, manufactured by Tomy Kogyo Co., Ltd.), and the residue was redispersed in 0.25 mL of ultrapure water to obtain an aqueous dispersion of three-layer supported fluorescent silica particles of silica particle/fluorescent layer (BSA-Au NCs)/polymer layer (Cs)/fluorescent layer (GSH-Au NCs).

### [Fluorescence spectrum measurement]

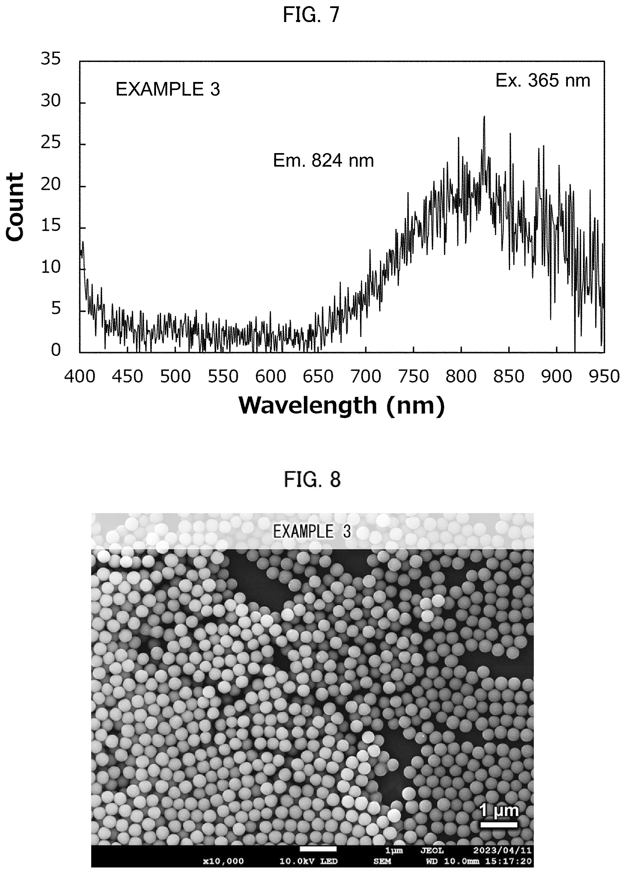

The fluorescence spectrum of the fluorescent particles of Example 3 with respect to 365 nm UV was measured by an absolute PL quantum yield measurement system (C11347-01, manufactured by Hamamatsu Photonics K.K.). The measurement result of the fluorescence spectrum is shown in Fig. 7. As shown in Fig. 7, a fluorescence peak of the fluorescent particles of Example 3 was observed in the near-infrared light region at 824 nm.

### [Observation of particle shape]

The shape of the fluorescent particles of Example 3 was observed using an electron microscope. An SEM image was acquired using an electron microscope JSM-7800F Prime (JEOL Ltd.) under the conditions of an acceleration voltage of 10.0 kV, a WD of 10.0 mm, and a magnification of 10,000x. The acquired SEM image is shown in Fig. 8. As shown in Fig. 8, the fluorescent particles of Example 3 had a spherical shape similar to the raw material silica particles, and also, since each particle shown in Fig. 8 was sufficiently discrete, it was also suggested that the fluorescent particles had superior dispersibility.

### [Reference Example 3] Preparation of single-layer supported fluorescent silica particles (NH₂-silica/GSH-Au NCs particles)

As core particles, silica particles (NH₂-silica) (average particle diameter: 325 nm, surface functional group: amino group) were used. 5 mL of an aqueous dispersion of silica particles (particle concentration: 0.01 g/mL) was added to a 20 cc screw-cap vial. Next, 1 mL of an aqueous dispersion of GSH-coated gold nanoclusters (gold atom concentration: 5 mM, prepared by the same method as in Example 3) was added to the aqueous dispersion of silica particles, and the mixture was stirred for 5 hours at room temperature using a magnetic stirrer (HERACLES-16G, manufactured by Koike Precision Instruments Inc.). The resulting solution was washed three times (3000 g, 3 min, 4 °C) using a centrifuge (MDX-110, manufactured by Tomy Kogyo Co., Ltd.), and the residue was redispersed in 3 mL of ultrapure water to obtain an aqueous dispersion of single-layer supported fluorescent particles of silica particle/fluorescent layer (GSH-Au NCs).

### [Example 4] Preparation of two-layer supported fluorescent silica particles (NH₂-silica/GSH-Au NCs/CS particles)

1.5 mL of a chitosan (CS) (FUJIFILM Wako Pure Chemical Corporation) solution (chitosan concentration: 1 mg/mL, 10 v/v% aqueous acetic acid solution) was prepared and added to 1.5 mL of the aqueous dispersion obtained in Reference Example 3. The mixture was stirred for 5 hours at room temperature using a magnetic stirrer (HERACLES-16G, manufactured by Koike Precision Instruments Inc.). The resulting solution was washed three times (3000 g, 3 min, 4 °C) using a centrifuge (MDX-110, manufactured by Tomy Kogyo Co., Ltd.), and the residue was redispersed in 3 mL of ultrapure water to obtain an aqueous dispersion of two-layer supported fluorescent silica particles of silica particle/fluorescent layer (GSH-Au NCs)/polymer layer (CS).

### [Example 5] Preparation of three-layer supported fluorescent silica particles (NH₂-silica/GSH-Au NCs/CS/GSH-AuAg NCs particles)

An aqueous solution was prepared to have final concentrations of 5.8 mM GSH, 3.5 mM chloroauric acid (HAuCl₄), 0.9 mM silver nitrate (AgNO₃), and 70 mM sodium hydroxide (NaOH). Next, the aqueous solution was stirred overnight at 37 °C to obtain an aqueous dispersion of GSH-coated gold-silver alloy nanoclusters (GSH-AuAg-NCs) (gold atom concentration: 5 mM).

2 mL of the aqueous dispersion of GSH-coated gold-silver alloy nanoclusters (gold atom concentration: 5.0 mM) was added to the aqueous dispersion obtained in Example 4. The mixture was stirred for 5 hours at room temperature using a magnetic stirrer (HERACLES-16G, manufactured by Koike Precision Instruments Inc.). The resulting solution was washed three times (3000 g, 3 min, 4 °C) using a centrifuge (MDX-110, manufactured by Tomy Kogyo Co., Ltd.), and the residue was redispersed in 3 mL of ultrapure water to obtain an aqueous dispersion of three-layer supported fluorescent silica particles of silica particle/fluorescent layer (GSH-Au NCs)/polymer layer (CS)/fluorescent layer (GSH-AuAg-NCs).

### [Fluorescence spectrum measurement]

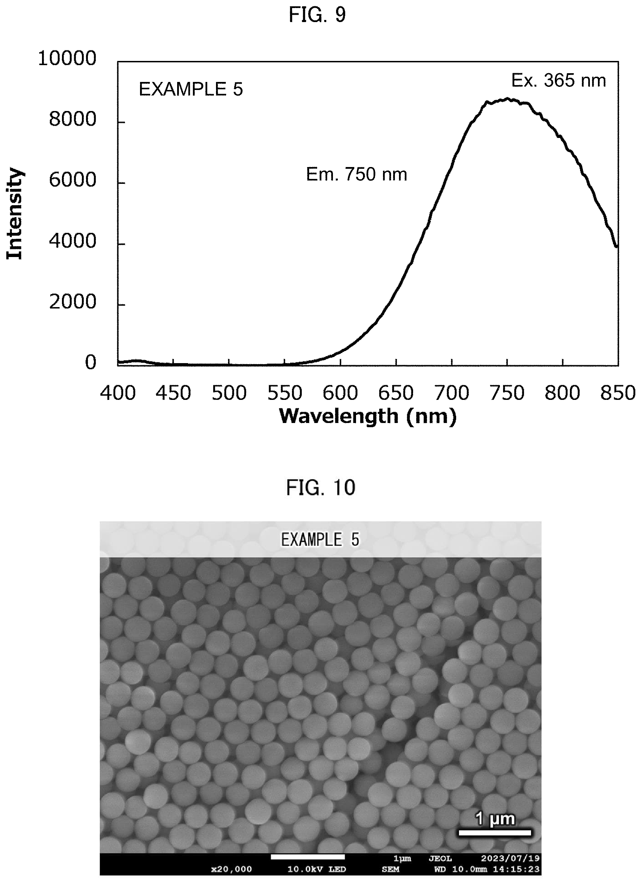

The fluorescence spectrum of the fluorescent particles of Example 5 with respect to 365 nm UV was measured by a microplate reader (Infinite (registered trademark) M200 PRO, manufactured by Tecan Group Ltd.). The measurement result of the fluorescence spectrum is shown in Fig. 9. As shown in Fig. 9, a fluorescence peak of the fluorescent particles of Example 5 was observed in the near-infrared light region at 750 nm.

### [Observation of particle shape]

The shape of the fluorescent particles of Example 5 was observed using an electron microscope. An SEM image was acquired using an electron microscope JSM-7800F Prime (JEOL Ltd.) under the conditions of an acceleration voltage of 10.0 kV, a WD of 10.0 mm, and a magnification of 20,000x. The acquired SEM image is shown in Fig. 10. As shown in Fig. 10, the fluorescent particles of Example 3 had a spherical shape similar to the raw material silica particles, and also, since each particle shown in Fig. 10 was sufficiently discrete, it was also suggested that the fluorescent particles had superior dispersibility.

### [Particle diameter distribution determination]

The particle diameter distribution of the fluorescent particles of Example 5 was determined using a dynamic light scattering (DLS) method. The determination was performed using a zeta potential/particle size/molecular weight analyzer ELSZ-2000 (Otsuka Electronics Co., Ltd.). The measurement results (not illustrated) indicated that the fluorescent particles of Example 5 had an average particle diameter of 345.1 ± 34.8 nm. This value is close to the value of the average particle diameter of the raw material silica particles (325 nm), and this result also suggested that the fluorescent particles of Example 5 had superior dispersibility.

### Industrial Applicability

The present invention can be utilized in the field of diagnostics using immunochromatography and the like.

### Reference Signs List

- 10A, 10B: Core particle
- 11, 11A, 11B: Fluorescent layer
- 12: Polymer layer
- 13: Metal nanoclusters
- 100, 101: Fluorescent particle

## Claims

1. A fluorescent particle comprising a core particle charged with a first charge that is a positive charge or a negative charge, and
further comprising on the core particle:
at least one polymer layer that contains a polymer and is charged with a second charge that is a positive charge or a negative charge; and
at least one fluorescent layer that contains fluorescent metal nanoclusters and is charged with a third charge having a sign opposite to that of the second charge.

2. The fluorescent particle according to claim 1, wherein at least one of the at least one fluorescent layer is disposed outside an outermost layer of the at least one polymer layer.

3. The fluorescent particle according to claim 1, wherein at least one of the at least one polymer layer is disposed outside an outermost layer of the at least one fluorescent layer.

4. The fluorescent particle according to claim 1, wherein
the first charge is a charge having the same sign as the second charge, and
at least one of the at least one fluorescent layer is disposed between the core particle and the at least one polymer layer.

5. The fluorescent particle according to claim 4, wherein
the first charge is a positive charge, and
the core particle has, on a surface thereof, a functional group selected from the group consisting of an amine group and a phosphonium group.

6. The fluorescent particle according to claim 4 or 5, wherein the core particle is selected from the group consisting of a silica particle, a polymer particle, and a metal particle.

7. The fluorescent particle according to any one of claims 1 to 5, wherein the fluorescent particle has an average particle diameter of not less than 100 nm and not more than 100 µm.

8. The fluorescent particle according to any one of claims 1 to 5, wherein the polymer comprises chitosan.

9. The fluorescent particle according to any one of claims 1 to 5, wherein
the metal nanoclusters are coated with a coating agent, and
the coating agent is at least one selected from the group consisting of an amino acid, a peptide, and a protein.

10. A method for producing a fluorescent particle, comprising:
at least one polymer layer formation step of forming, on a core particle charged with a first charge that is a positive charge or a negative charge, at least one polymer layer that contains a polymer and is charged with a second charge that is a positive charge or a negative charge; and
at least one fluorescent layer formation step of forming, on the core particle, at least one fluorescent layer that contains fluorescent metal nanoclusters and is charged with a third charge having a sign opposite to that of the second charge.

11. The method according to claim 10, wherein
the first charge is a charge having the same sign as the second charge, and
at least one of the at least one fluorescent layer formation step is performed before the at least one polymer layer formation step.

12. The method according to claim 11, further comprising, before the at least one polymer layer formation step and the at least one fluorescent layer formation step, a charging step of obtaining the core particle by a charging treatment.

13. The method according to any one of claims 10 to 12, wherein the at least one polymer layer formation step and the at least one fluorescent layer formation step are each performed at not less than 0 °C and not more than 100 °C.
